# EUROPEAN PATENT SPECIFICATION

(11) **EP 1 847 228 B1**
(45) Date of publication and mention of the grant of the patent: **05.08.2009**
(21) Application number: 07251649.5
(22) Date of filing: 19.04.2007
(51) Int. Cl.: A61B 17/74, A61B 17/80, A61B 17/04

(54) **Fixation plate with multifunctional holes**
Befestigungsplatte mit Multifunktionslöchern
Plaque de fixation dotée de trous multifonction

(30) Priority: 21.04.2006 US 379646
(43) Date of publication of application: 24.10.2007
(73) Proprietor: DePuy Products, Inc., Warsaw, IN 46581 (US)
(72) Inventor: Bremer, Christopher Kelly, Warsaw, IN 46582 (US); Orbay, Jorge L., Coral Gables, FL 33156 (US); Cavallazzi, Cesare, Miramar, FL 33027 (US); McBarak, Edward, Miami, FL 33172 (US)
(74) Representative: Gover, Richard Paul

(56) References cited:
- WO-A-02/096309
- US-A1- 2005 182 405
- US-A1- 2005 234 458

## Description

This invention relates to a fracture fixation plate, in particular which can be used with K-wires and/or suture material.

Fracture fixation plates are common in the industry. Such plates are use to bridge across fractures, stabilizing fragments for healing. Fixation plates may be in the form of fragment plates which are generally used along the diaphysis of long bones or anatomically specific metaphyseal plates which are contoured for the articulating end of long bones.
The plates are generally provided with a plurality of screw holes at which the plates can be secured to the bone with screws or other fasteners. The holes may be locking holes, non-locking holes, combination holes (which can be locking or non-locking depending upon the fastener which is used), and a combination of any of the above.

It is common for fixation plates to include K-wire holes. Stiff K-wires are often used to temporarily secure a plate to the bone prior to and during fixation by the fasteners. The K-wires are then removed.

In addition, it is known to provide fixed angle K-wire holes that have a cylindrical bore and which are sized to closely fit with K-wires so as to guide K-wires in a predefined angular orientation. Such orientation is closely related to the angle of one or more of the fasteners such that the path of the inserted K-wires anticipates the path of the fasteners. Then, when viewing the plate and inserted K-wires under fluoroscopy, the surgeon is provided with visual indication of whether the fasteners, once inserted, will be properly aligned with the anatomy. Such a technique is disclosed in US-A-2005/0065524.

Furthermore, depending upon the surgical procedure, the surgeon may need to incise the insertions of certain muscles to facilitate reduction of the fractured bone and to provide access for attachment of the plate to the bone. For example, in a procedure to fixate a proximal humeral fracture, the deltoid and pectoralis muscle insertions are commonly incised and after fixing the plate to the bone, the deltoid and pectoralis tendons may be fixed to the plate to reconstruct the musculature. As such, it is known to provide a plate with suture holes for receiving a suture needle and suture material, e.g., cord or cable. Suture holes are designed differently than K-wires holes. Rather than have structure for fixed angle guidance, the suture holes are heavily chamfered for prevent damage to the suture material.

Thus, fixation plates are often replete with holes with dedicated functionality. Such holes crowd the plate surface, often making it difficult for a surgeon to identify which holes are to be used for which purpose. In addition, there may not be sufficient space on the plate to place the holes at the optimal locations for the desired purposes. Furthermore, the number of holes dedicated to each function may result in a plate that is weaker than desired for a particular application.

US-A-2005/0261688 discloses a plate having holes that are indicated to be for either guide wires or suture, thereby reducing the total number of holes. However, there is no disclosure that the holes are intended to guide stiff wires at a fixed angle. In addition, the design of the holes does not permit a suture needle to be inserted between the bone and the bone contacting surface of the plate after the plate is positioned against or attached to the bone. Suture holes which permit suture needle passage after the plate is fixed to the bone generally include a bottom clearance for the needle, as in the product sold under the trade mark Humeral SuturePlate by Arthrex Inc.

US-2005/0182405 discloses a fracture fixation system for a fracture of a head portion of a long bone which has subchondral bone defining a convex articular surface. The system includes a plate element positionable on the long bone substantially opposite the head portion of the long bone and on a first side of the fracture, and a post element extending from the plate and into the head portion and across the fracture. A support structure for supporting the subchondral bone of the articular surface is coupled to the post. After the fracture is reduced and prior to drilling a hole for the post, the surgeon drills K-wires through alignment holes on the head portion of the plates to temporarily fix the orientation of the head of the plate to the head of the humerus. Once the posts are implanted, the K-wires can be removed. The head portion includes a lower proximal recess and a plurality of suture guides with holes thereabout. The recess raises the suture guides off the surface of the bone to allow the surgeon to pass a needle with suture material through the suture guides and between the plate and the bone to permit tendon and bone fragments to be sutured to the plate.

The scope of the present invention is defined by the appended claims.

The present invention provides a bone plate for internal fixation of a bone fracture includes top and bottom surfaces and an edge. At least one multifunctional hole is provided along the edge and communicates between the top and bottom surfaces. The hole generally includes a cylindrical portion for closely receiving and guiding a stiff K-wire in a fixed angle. The hole also includes a lateral channel or recess formed into the bottom surface and an edge of the plate such that a curved suture needle and attached suture material may be passed through the hole and out of the side of the plate even when the plate is fixed to the bone. A plurality of multifunctional holes may be arranged along the edges of the plate, either closely spaced, e.g. in a metaphyseal plate, or spaced apart along sides of a diaphyseal plate. If arranged in a staggered manner along opposed sides, the plate maintains its longitudinal and torsional stiffness, whereas if the multifunctional holes are in an opposing arrangement, the plate develops areas of decreased stiffness at which the plate can bend to conform to the anatomy when coupled to the bone.

Accordingly, in one aspect, the invention provides a bone plate for fixation of a bone fracture, comprising a bone plate for internal fixation of a bone fracture, the plate having top and bottom surfaces and at least one edge, in which the plate has a plurality of screw holes extending between the top and bottom surfaces for receiving fixators to couple the plate to the bone, and in which the plate has at least one multifunctional hole extending through it, the multifunctional hole including a wire guide portion which is structured to guide a stiff wire at a substantially fixed angle relative to the plate and a lateral recess extending from the guide portion to an adjacent edge, the lateral recess open to the bottom surface of the plate.

In another aspect, the invention provides a bone plate for internal fixation of a bone fracture, for use with a stiff wire and a suture needle, the plate including top and bottom surfaces and a peripheral edge thereabout, and having a plurality of screw holes extending between the top and bottom surfaces for receiving fixators to couple the plate to the bone, the plate including:
a first wire guide structured to guide the stiff wire at a substantially fixed angle relative to the plate,
a second wire guide structured to guide the stiff wire at a substantially fixed angle relative to the plate, each of the first and second wire guides having an entry and an exit, and
a recess extending from the exits of the first and second guides to the peripheral edge,
in which the suture needle may be passed through either of the first and second wire guides and exit laterally of the plate through the recess after the bottom surface of the plate has been coupled substantially flush against the bone.

In a further aspect, the invention provides a bone plate for fixation of a bone fracture, comprising a bone plate for internal fracture fixation, the plate including at least one multifunctional hole including both guide means for guiding a stiff wire at a substantially fixed angle and needle exit means for exiting a curved suture needle out of the multifunctional hole and out of a periphery of the plate after the plate has been coupled substantially flush against the bone.

In another aspect, the invention provides a bone plate for fixation of a bone fracture, comprising a bone plate for internal fixation of a bone fracture, the plate including top and bottom surfaces and at least one edge thereabout, in which the plate defines a plurality of screw holes extending between the top and bottom surfaces for receiving fixators to couple the plate to the bone, and in which the plate has at least one multifunctional hole, the multifunctional hole including a wire guide portion which is structured to receive a stiff wire through the top and bottom surfaces of the plate and a lateral recess extending from the guide portion to an adjacent edge, the lateral recess open to the bottom surface of the plate.

The fracture fixation plate of the invention has a multifunctional hole which can accommodate a K-wire at a fixed angle and which allows a suture needle to be inserted through the hole even after the plate has been fixed to the bone.

The plate has a multifunctional hole that reduces the total number of holes required in a plate, thereby maintaining desired plate strength and allowing optimal placement of the holes within the plate.

The multifunctional holes in the plate in an arrangement that minimise loss of longitudinal and torsional stiffness of the plate.

The multifunctional holes in the plate can be provided in an arrangement that increases flexibility of the plate at specific locations.

The multifunctional hole configuration can minimise stress concentration to sutures extending through it.

The multifunctional hole feature of the invention can be provided in metaphyseal plates or in diaphyseal plates.

Embodiments of the invention will now be described by way of example with reference to the accompanying drawings, in which:
Fig. 1 is a top view of a first embodiment of a bone plate including a multifunctional hole according the invention;
Fig. 2 is a side elevation view of the first embodiment of the bone plate;
Fig. 3 is a bottom view of the first embodiment of the bone plate;
Fig. 4 is an enlarged top view of a multifunctional hole in a bone plate;
Fig. 5 is an enlarged side elevation of the multifunctional hole;
Fig. 6 is an enlarged bottom view of the multifunctional hole;
Fig. 7 is a section view across line 7-7 in Fig. 1;
Fig. 8 is a view similar to Fig. 7, shown with the bone plate positioned on bone and a K-wire held within a fixed-angle with the multifunctional hole;
Fig. 9 is a view similar to Fig. 7, shown with the bone plate positioned on bone and a suture needle extending through the multifunctional hole;
Fig. 10 is a top view of a second embodiment of a bone plate with a multifunctional hole;
Fig. 11 is a bottom view of the second embodiment of the bone plate;
Fig. 12 is a section view across line 12-12 in Fig. 10;
Fig. 13 is a perspective view of a third embodiment of a bone plate including a multifunctional hole according the invention;
Fig. 14 is a top view of the third embodiment of a bone plate;
Fig. 15 is a longitudinal section view of the third embodiment of the bone plate; and
Fig. 16 is a bottom view of the third embodiment of the bone plate.

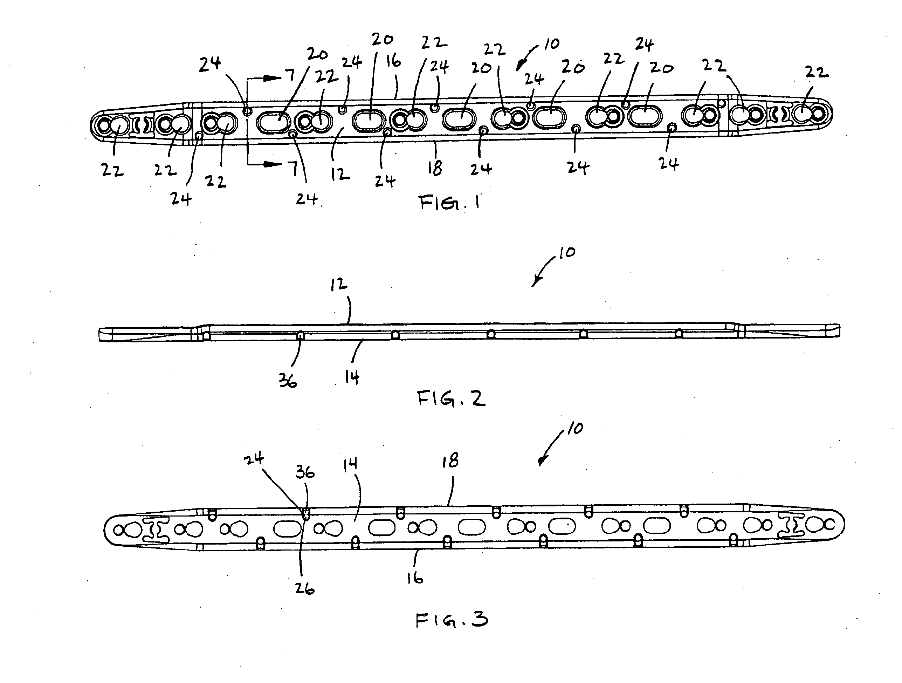

Referring to the drawings, Figs. 1 to 3 show a first embodiment of bone plate 10 for internal fixation of a bone fracture is shown. The plate is an elongate diaphyseal plate for fractures along a shaft portion of a long bone. The plate includes top and bottom surfaces 12, 14 and edges 16, 18 thereabout. Between the top and bottom (bone contacting) surfaces and along the length of the plate a plurality of screw holes 20, 22 are provided. Such screw holes may be variable angle, fixed angle, or combination variable and fixed angle screw holes. Commonly known fixators, such as, e.g., fixed angle screws, variable angle screws, locking screws, and locking pegs, may be provided for use through the holes 20, 22.

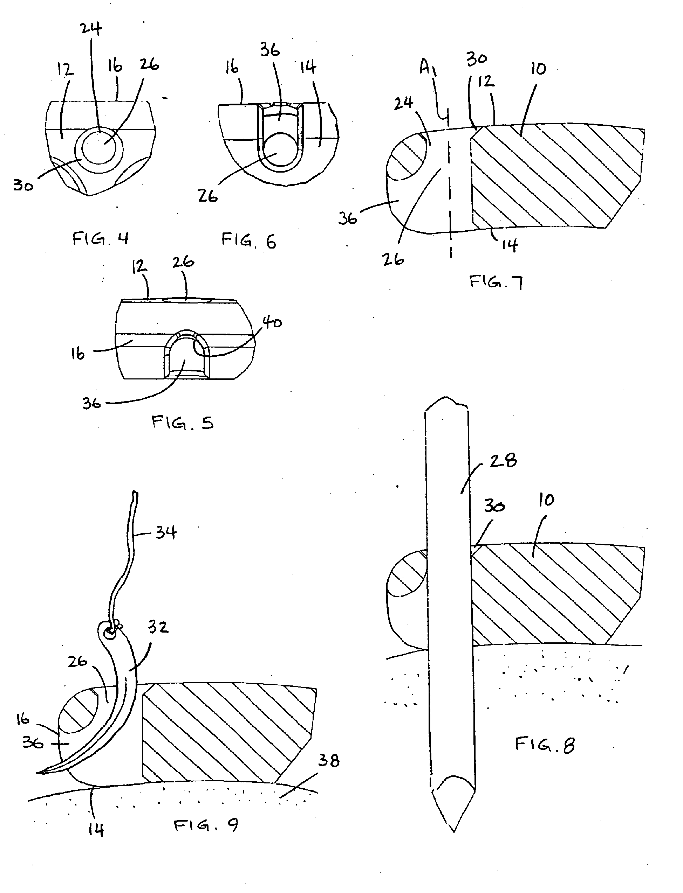

Referring to Figs. 2 through 9, a plurality of multifunctional holes 24 for both K-wire and suture needle/suture material is also provided along the length of the plate adjacent the edges 16, 18 and communicate between the top and bottom surfaces 12, 14. Each multifunctional hole 24 generally includes a cylindrical portion 26 sized for closely receiving and guiding substantially along fixed axis A1 (e.g., ±3°) an appropriately sized stiff K-wire 28 (Fig. 8). The axis A1 is generally approximately normal to the top and/or bottom surfaces 12, 14 of the plate. As an alternative, portion 26 can be non-cylindrical, but otherwise shaped for substantially fixed-angle guidance of the K-wire along axis A1. For example, the portion 26 may be provided with a regular polygonal cross-sectional (hexagonal, octagonal, etc.) which also guides the K-wire in a substantially fixed angle, as described. The cylindrical portion 26 has a circular upper opening 30 which is slightly flared at its entry to facilitate introduction of the K-wire 28 (Fig. 8) or suture needle 32 (Fig. 9, discussed below) and prevent stress concentration on suture material 34. The multifunctional hole 24 also includes a lateral channel (or lower recessed portion) 36 (i.e., directed toward the adjacent edge 16 of the plate) formed into the bottom surface 14 and the adjacent edge 16 of the plate such that a curved suture needle 32 and attached suture material 34 may be passed through the hole and laterally out of the side of the plate even when the plate is fixed to bone 38. The lateral channel 36 preferably assumes approximately half the height of the plate. The surface of the lateral channel 36 is defined by the inner surface of a toroidal section; i.e., suture guide surface 40 is concave across its width, as shown at the exit of the channel 36 in Fig. 5. In addition, all surfaces along the suture channel are broken or radiussed. Thus, when suture material 34 is passed through the plate, stress concentrations on the suture material is limited and the likelihood of suture material breakage is minimized.

Referring back to Figs. 1 and 3, a plurality of the multifunctional holes 24 may be arranged adjacent the edges 16, 18 of the plate 10 in staggered arrangement. The alternating arrangement substantially maintains the longitudinal and torsional stiffness of the plate.

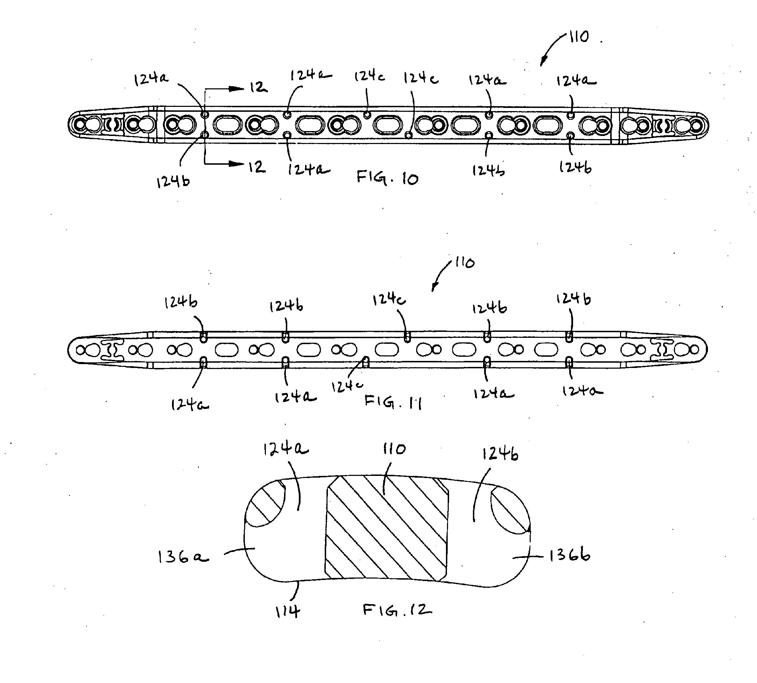

Turning now to Figs. 10 to 12, according to second embodiment of the invention, it is also recognized that the multifunctional holes 124a, 124b may be spaced apart along the fixation plate 110 in an arrangement of opposed pairs. At each opposed pair of multifunctional holes 124a, 124b, the plate develops areas of decreased longitudinal stiffness as a result of the opposed lateral recesses 136a, 136b at the bottom surface 114 of the plate. By way of example, Fig. 12 is a view across an area of decreased longitudinal stiffness. The decreased stiffness at such areas allows the plate to bend to conform to the shape of the bone when the plate is attached to the bone with fixators. Such plate may additionally include staggered holes 124c as well.

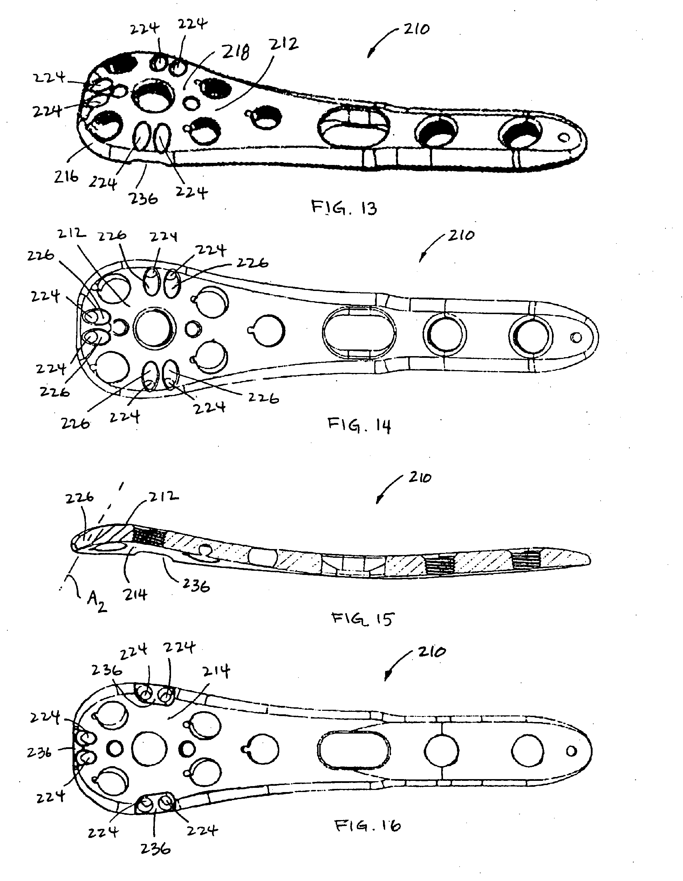

Figs. 13 to 16 show a third embodiment of the invention. The specific plate 210 shown, by way of example, is a metaphyseal plate for fixation of humeral fractures. The plate includes top and bottom surfaces 212, 214. In plate 210, three closely spaced pairs of multifunctional holes 224 are shown located around the peripheral edge 216 of the head portion 218 of the plate 210. The cylindrical K-wire guiding portion 226 of a multifunctional hole is oriented at an oblique angle along axis A2 relative to the top and bottom surfaces 212, 214 of the plate. At the bottom surface 214 of the plate a wide lateral channel or recess 236 is provided and encompasses the exits of both K-wire guiding portions 226 of the holes.

## Claims

1. A bone plate (10) for fixation of a bone fracture, comprising a bone plate (10) for internal fixation of a bone fracture, the plate (10) including top (12) and bottom (14) surfaces and at least one edge (16, 18) thereabout, in which the plate (10) has a plurality of screw holes (20, 22) extending between the top and bottom surfaces (12, 14) for receiving fixators to couple the plate (10) to the bone, and in which the plate (10) has at least one multifunctional hole (24) extending through it, **characterised in that** the multifunctional hole (24) includes a wire guide portion (26) which is structured to guide a stiff wire (28) at a substantially fixed angle relative to the plate (10) and a lateral recess (36) extending from the guide portion (26) to an adjacent edge (16, 18), the lateral recess (36) open to the bottom surface (14) of the plate (10).

2. A bone plate (10) according to claim 1, in which the guide portion (26) is cylindrical.

3. A bone plate (10) according to claim 1, in which the guide portion (26) defines an axis that is normal to at least one of the top and bottom surfaces (12, 14) of the plate (10).

4. A bone plate (10) according to claim 1, in which the guide portion (26) defines an axis that is oblique relative to at least one of the top and bottom surfaces (12, 14) of the plate (10).

5. A bone plate (10) according to claim 1, in which the guide portion (26) has a flared opening (30) at the top surface (12).

6. A bone plate (10) according to claim 1, in which the plate (10) has a height, and at the adjacent edge (16, 18) the lateral recess (36) assumes approximately half the height of the plate (10).

7. A bone plate (10) according to claim 1, in which the lateral recess (36) includes a curved suture guide surface (40).

8. A bone plate (10) according to claim 1, in which the exit of the lateral recess (36) at the adjacent edge (16, 18) is concavely curved.

9. A bone plate (10) according to claim 1, which includes a plurality of the multifunctional holes (24), spaced apart along the length of the plate (10).

10. A bone plate (10) according to claim 1, which includes a plurality of the multifunctional holes (24), in a staggered arrangement the length of the plate.

11. A bone plate (110) according to claim 1, which includes a plurality of the multifunctional holes (124) which are arranged in pairs, with the holes (124) of each pair located along respective edges of the plate (110), directly across from one another.

12. A bone plate (10) according to claim 1, in which the plate is a diaphyseal plate or a metaphyseal plate.

13. A bone plate (210) according to claim 1 for use with a stiff wire and a suture needle, the plate (210) including two multifunctional holes (224), each wire guide (226) having an entry and an exit, and a recess (236) extending from the exits of the first and second guides (226) to the peripheral edge; wherein the suture needle may be passed through either of the first and second wire guides (226) and exit laterally of the plate (210) through the recess (236) after the bottom surface of the plate (210) has been coupled substantially flush against the bone.

14. A bone plate (210) according to claim 13, in which at least one of the first and second wire guides (226) defines a fixed angle axis for the stiff wire that is oblique relative to one of the top and bottom surfaces of the plate (210).

15. A bone plate (210) according to claim 13, in which the plate (210) is a metaphyseal plate including a head portion and the first and second wire guides (226) and recess (236) are provided together in the head portion.

16. A bone plate (210) according to claim 13, in which a plurality of combinations of the first and second wire guides (226) and recess (236) are spaced apart about a periphery of the head portion.

## Patentansprüche

1. Knochenplatte (10) zum Fixieren einer Knochenfraktur, mit einer Knochenplatte (10) zum internen Fixieren einer Knochenfraktur, wobei die Platte (10) eine obere (12) und eine untere (14) Fläche und wenigstens eine umlaufende Kante (16, 18) umfasst, wobei die Platte (10) eine Vielzahl von Schraubenlöchern (20, 22) hat, die sich zwischen der oberen und der unteren Fläche (12, 14) zum Aufnehmen von Befestigungselementen erstrecken, um die Platte (10) an den Knochen zu koppeln, und wobei die Platte (10) wenigstens ein durchgängiges multifunktionales Loch (24) hat, **dadurch gekennzeichnet, dass** das multifunktionale Loch (24) einen Drahtführungsbereich (26), der so strukturiert ist, dass er einen steifen Draht (28) unter einem im Wesentlichen festen Winkel relativ zu der Platte (10) führt, und eine seitliche Ausnehmung (36), die sich von dem Führungsbereich (26) zu einer benachbart liegenden Kante (16, 18) erstreckt, wobei die seitliche Aufnehmung (36) zu der unteren Fläche (14) der Platte (10) offen ist, umfasst.

2. Knochenplatte (10) nach Anspruch 1, bei der der Führungsbereich (26) zylindrisch ist.

3. Knochenplatte (10) nach Anspruch 1, bei der der Führungsbereich (26) eine Achse definiert, die zu wenigstens einer, der oberen oder der unteren Fläche (12, 14) der Platte (10) normal ist.

4. Knochenplatte (10) nach Anspruch 1, bei der der Führungsbereich (26) eine Achse definiert, die in Bezug auf wenigstens eine, die obere oder die untere Fläche (12, 14) der Platte (10) schräg verläuft.

5. Knochenplatte (10) nach Anspruch 1, bei der der Führungsbereich (26) an der oberen Fläche (12) eine aufgeweitete Öffnung (30) hat.

6. Knochenplatte (10) nach Anspruch 1, wobei die Platte (10) eine Höhe hat und seitliche Ausnehmung (36) an der benachbart liegenden Kante (16, 18) ungefähr die Hälfte der Höhe der Platte (10) einnimmt.

7. Knochenplatte (10) nach Anspruch 1, bei der die seitliche Ausnehmung (36) eine gekrümmte Fadenführungsfläche (40) umfasst.

8. Knochenplatte (10) nach Anspruch 1, bei der der Ausgang der seitlichen Öffnung (36) an der benachbart liegenden Kante (16, 18) konkav gekrümmt ist.

9. Knochenplatte (10) nach Anspruch 1, die eine Vielzahl der multifunktionalen Löcher (24) umfasst, welche entlang der Länge der Platte (10) beabstandet sind.

10. Knochenplatte (10) nach Anspruch 1, die eine Vielzahl der multifunktionalen Löcher (24) in einer gestuften Anordnung über die Länge der Platte umfasst.

11. Knochenplatte (110) nach Anspruch 1, die eine Vielzahl der multifunktionalen Löcher (124) umfasst, welche in Paaren angeordnet sind, wobei die Löcher (124) jedes Paares entlang jeweiliger Kanten der Platte (110) einander direkt gegenüber angeordnet sind.

12. Knochenplatte (10) nach Anspruch 1, wobei die Platte eine Diaphysenplatte oder eine Metaphysenplatte ist.

13. Knochenplatte (210) nach Anspruch 1 zur Verwendung mit einem steifen Draht und einer Fadennadel, wobei die Platte (210) zwei multifunktionale Löcher (224) umfasst, wobei jede Drahtführung (226) einen Eingang und einen Ausgang hat und sich eine Ausnehmung (236) von den Ausgängen der ersten und der zweiten Führung (226) zur Umfangskante erstreckt; wobei die Fadennadel durch entweder die erste oder die zweite Drahtführung (226) geführt werden und seitlich aus der Platte (210) durch die Ausnehmung (236) austreten kann, nachdem die untere Fläche der Platte (210) im Wesentlichen bündig angekoppelt ist.

14. Knochenplatte (210) nach Anspruch 13, bei der wenigstens eine, die erste oder die zweite Drahtführung (226), eine feste Winkelachse für den steifen Draht definiert, die in Bezug auf eine, die obere oder die untere Fläche der Platte (210) schräg verläuft.

15. Knochenplatte (210) nach Anspruch 13, wobei die Platte (210) eine Metaphysenplatte ist, die einen Kopfbereich umfasst, und die erste und die zweite Drahtführung (226) und die Ausnehmung (236) zusammen in dem Kopfbereich vorgesehen sind.

16. Knochenplatte (210) nach Anspruch 13, bei der eine Vielzahl von Kombinationen aus der ersten und der zweiten Drahtführung (226) und der Ausnehmung (236) um einen Umfangsbereich des Kopfbereiches beabstandet ist.

## Revendications

1. Plaque pour os (10) destinée à la fixation d'une fracture osseuse, comprenant une plaque pour os (10) pour une fixation interne d'une fracture osseuse, la plaque (10) comprenant une surface supérieure (12) et une surface inférieure (14) et au moins un bord (16, 18) situé autour, dans laquelle la plaque (10) présente une pluralité de trous de vis (20, 22) qui s'étendent entre les surfaces supérieure et inférieure (12, 14), destinés à recevoir des dispositifs de fixation de manière à relier la plaque (10) à l'os, et dans laquelle la plaque (10) présente au moins un trou multifonction (24) qui s'étend à travers celle-ci, **caractérisée en ce que** le trou multifonction (24) comprend une partie de guidage de fil (26) qui est structurée de manière à guider un fil rigide (28) sous un angle sensiblement fixe par rapport à la plaque (10), et un retrait latéral (36) qui s'étend à partir de la partie de guidage (26) vers un bord adjacent (16, 18), le retrait latéral (36) étant ouvert vers la surface inférieure (14) de la plaque (10).

2. Plaque pour os (10) selon la revendication 1, dans laquelle la partie de guidage (26) est cylindrique.

3. Plaque pour os (10) selon la revendication 1, dans laquelle la partie de guidage (26) définit un axe qui est perpendiculaire à l'une au moins des surfaces supérieure et inférieure (12, 14) de la plaque (10).

4. Plaque pour os (10) selon la revendication 1, dans laquelle la partie de guidage (26) définit un axe qui est oblique par rapport à l'une au moins des surfaces supérieure et inférieure (12, 14) de la plaque (10).

5. Plaque pour os (10) selon la revendication 1, dans laquelle la partie de guidage (26) présente une ouverture évasée (30) au niveau de la surface supérieure (12).

6. Plaque pour os (10) selon la revendication 1, dans laquelle la plaque (10) présente une hauteur, et au niveau du bord adjacent (16, 18) le retrait latéral (36) fait approximativement la moitié de la hauteur de la plaque (10).

7. Plaque pour os (10) selon la revendication 1, dans laquelle le retrait latéral (36) comprend une surface de guidage de suture incurvée (40).

8. Plaque pour os (10) selon la revendication 1, dans laquelle la sortie du retrait latéral (36) au niveau du bord adjacent (16, 18), est incurvée de manière concave.

9. Plaque pour os (10) selon la revendication 1, qui comprend une pluralité de trous multifonctions (24), espacés sur la longueur de la plaque (10).

10. Plaque pour os (10) selon la revendication 1, qui comprend une pluralité de trous multifonctions (24), disposés de manière décalée sur la longueur de la plaque.

11. Plaque pour os (110) selon la revendication 1, qui comprend une pluralité de trous multifonctions (124) qui sont disposés en paires, les trous (124) de chaque paire étant situés le long des bords respectifs de la plaque (110), directement à travers les uns des autres.

12. Plaque pour os (10) selon la revendication 1, dans laquelle la plaque est une plaque diaphysaire ou une plaque métaphysaire.

13. Plaque pour os (210) selon la revendication 1, destinée à être utilisé avec un fil rigide et une aiguille de suture, la plaque (210) comprenant deux trous multifonctions (224), chaque dispositif de guidage de fil (226) présentant une entrée et une sortie, et un retrait (236) qui s'étend à partir des sorties des premier et deuxième dispositifs de guidage (226) vers le bord périphérique ; dans laquelle l'aiguille de suture peut être passée à travers le premier ou le deuxième dispositif de guidage de fil (226) et sortir de manière latérale de la plaque (210) à travers le retrait (236), une fois que la surface inférieure de la plaque (210) a été reliée sensiblement de niveau contre l'os.

14. Plaque pour os (210) selon la revendication 13, dans laquelle l'un au moins des premier et deuxième dispositifs de guidage de fil (226), définit un axe d'angle fixe pour le fil rigide, qui est oblique par rapport à l'une des surfaces supérieure et inférieure de la plaque (210).

15. Plaque pour os (210) selon la revendication 13, dans laquelle la plaque (210) est une plaque métaphysaire qui comprend une partie principale, et les premier et deuxième dispositifs de guidage de fil (226) et la cavité (236) sont disposés ensemble dans la partie principale.

16. Plaque pour os (210) selon la revendication 13, dans laquelle une pluralité de combinaisons de premiers et de deuxièmes dispositifs de guidage de fil (226) et de retraits (236), sont espacés autour de la périphérie de la partie principale.
